# EUROPEAN PATENT APPLICATION

(11) **EP 4 445 903 A1**
(43) Date of publication of application: **16.10.2024**
(21) Application number: 24168224.4
(22) Date of filing: 03.04.2024
(51) Int. Cl.: A61K 31/728, A61K 33/00, A61P 15/02

(54) **ASSOCIATION OF OZONE IN GASEOUS FORM AND HYALURONIC ACID FOR THE TREATMENT OF VAGINAL DISORDERS**

(30) Priority: 04.04.2023 IT 202300006579
(71) Applicant: Caress Flow S.r.l., 40050 Argelato (BO) (IT)
(72) Inventor: Montanari, Federico, 40050 Argelato BO (IT); Venturi, Flavio, 40050 Argelato BO (IT); Garoia, Flavio, 40050 Argelato BO (IT)
(74) Representative: Perani & Partners S.p.A.

(57) **Abstract**

The present invention relates to an association comprising: a first oxidising and conditioning active ingredient consisting of ozone in gaseous form and hyaluronic acid, for use in the topical treatment of vaginal disorders or pathological conditions.

## Description

### FIELD OF THE INVENTION

The present patent application concerns an association of two active substances, namely ozone in gaseous form and hyaluronic acid, for use in the treatment of vaginal disorders or pathological conditions.

### STATE OF THE ART

There are several disorders or diseases or vaginal pathological conditions that afflict women, especially during and after menopause, but not only.

Common disorders or diseases or pathological conditions affecting the vagina include, for example, vulvovaginal atrophy, which may or may not be associated with vaginal dryness.

Vulvovaginal atrophy (VVA) is a chronic pathological condition characterised by the thinning of the vaginal mucosa, with a consequent reduction in vascularity, elasticity and degree of hydration of the vaginal mucosa. In addition, VVA is characterised by an increase in vaginal pH associated with a reduction in lactobacilli, the "good" bacteria that protect the flora of the vaginal mucosa. Women who suffer from VVA have a series of symptoms typical of menopause (*Genitourinary Menopause Syndrome*) such as: vaginal dryness, itching, pain and bleeding during sexual intercourse.

The vaginal epithelium is a stratified squamous epithelium, which until menopause is lubricated, thick and rough. With the decrease in oestrogen levels, such as during menopause, the vaginal epithelium becomes thinner. A reduction in epithelial cells leads to a reduction in the exfoliation of the cells in the vagina, a process that normally has the function of releasing glycogen, hydrolysed into lactic acid by the action of lactobacilli, normally present in the vaginal flora. Without the release of glycogen and without the production of lactic acid, the pH of the vagina increases, resulting in an alteration of the local flora and a growth of harmful bacteria, which can cause infections and inflammation. VVA can in fact affect the lower urinary tract: the increased need and the sudden and uncontrollable stimulus to urinate are among the most common symptoms, as well as possible infections that can affect the entire area (cystitis).

Thinning and inflammation of the vaginal and/or vulvar wall contribute to reduced vaginal lubrication.

Vaginal dryness is a vaginal disorder linked to a lack of physiological local lubrication, the causes of which are numerous and include, for example: advancing age; hormonal changes; menopause; breastfeeding; stress; pathological conditions, such as diabetes, irritable bowel syndrome, chronic heart failure; iatrogenic causes, such as radiotherapy and chemotherapy or the use of antidepressants (D. Edwards and N. Panay. Treating vulvovaginal atrophy/genitourinary syndrome of menopause: how important is vaginal lubricant and moisturizer composition?. Taylor & Francis Climacteric. 2016 Mar 3; 19(2): 151-161.).

VVA often occurs under conditions of hypoestrogenism. In the pre-menopausal state, the oestradiol levels fluctuate from 10 to 800 pg/mL, depending on when measured during menstruation. In the post-menopausal state, oestradiol levels are typically below 30 pg/mL (Maire B. Mac Bride, MBBCh, Deborah J. Rhodes, MD, and Lynne T. Shuster, MD. Vulvovaginal Atrophy. Mayo Clin Proc. 2010 Jan; 85(1): 87-94).

VVA symptoms can be treated by using over-the-counter medications, where the choice of the therapy depends on the severity of the symptoms, the effectiveness of the therapy, and the patient's preferences.

Lubricants can relieve vaginal dryness during sexual intercourse, providing relief, albeit momentary, from vaginal dryness and dyspareunia.

In any case, the most effective treatments are those based on hormones, where the first-choice therapy is the administration of local oestrogens, including: oestriol, which is much lighter than oestradiol and can be used for years, promestriene and conjugated oestrogens. Local hormone therapy can solve the problems of genital dryness and atrophy in 85% of women after menopause, better if the treatment began immediately after the disappearance of menstruation. If there are also problems of dryness and less physical response also from the external genitals, a local testosterone ointment - galenic, on prescription - rekindles the physical response even more.

There is, however, a percentage of 10-12% of women, who cannot use oestrogen, not even locally, because they have been operated for breast cancer or adenocarcinoma of the ovary or uterus. To reduce dryness and pain in these subjects, it is possible to use vaginal hyaluronic acid, which has a restorative and antioxidant action; the vaginal laser, which is however much more expensive; or different creams that do not have the therapeutic impact of the hormones.

Hyaluronic acid is known to possess remarkable adhesive, moisturising and repairing properties of the vaginal mucosa. The use of hyaluronic acid in the treatment of vaginal dryness, through the use of lavages or ovules, is known to the state of the art. The use of vaginal lavages based on hyaluronic acid is to be considered as a therapeutic alternative to oestrogen-based treatments in alleviating disorders associated with vaginal dryness. (Chen J et al. "Evaluation of the efficacy and safety of hyaluronic acid vaginal gel to ease vaginal dryness: a multicenter, randomized, controlled, open-label, parallel-group, clinical trial" J Sex Med. 2013; 10; 1575-84).

On the other hand, there are known documents that disclose the use of ozone in gaseous form vaginally.

WO9810774A2 describes a method for treating a disease of a tissue of the body cavity, in which an ozone-containing gas (or "ozonated gas") can be introduced, for example, through the vaginal cavity. The same document also describes an apparatus, easily transportable, which incorporates an ozone generator, and which provides a flow of "ozonated gas"; the same apparatus is also suitable for controlling the flow of gas and the relative ozone content. According to the authors of WO9810774A2, the apparatus is: easy to handle; economical to manufacture; suitable for preventing the escape of gases or air containing a dangerous concentration of microorganisms or other contamination into the atmosphere.

UA93287 describes devices for the treatment of bacterial and viral diseases, vaginosis, inflammatory diseases of the lower genital organs, by using ozone or a liquid containing ozone.

On the other hand, documents are also known that disclose the use of ozone in oil-like systems ("oil carriers"), or in liposomal nanosolution technologies.

For example, WO2022186802A1 discloses liposomal ozone nanosolutions, capable of releasing ozone at the cellular level in a controlled manner, for the prevention and treatment of viral, bacterial or fungal diseases and for tissue wound healing. WO2022186802A1 describes that particulate systems with a coating of ozonated emulsifiers and/or non-ozonated emulsifiers can be obtained. For example, emulsifiers such as polysorbates and PEGs, can be ozonated and form stable nanoparticles.

Hyaluronic acid can be used for the coating of the particles in solution. WO2022186802A1 discloses that it is possible to increase the effectiveness of ozone emulsifiers by binding, individually or in combination, molecules, such as lipoproteins or hyaluronic acid, to the emulsifiers themselves, so as to obtain "coupled-structures" and direct them to the appropriate target organ. WO2022186802A1 does not specifically disclose the use of liposomal ozone nanosolutions for the treatment of vaginal atrophy and/or dryness.

EP3645016B1 discloses instead an association of *oxygen* in gaseous form and hyaluronic acid in aqueous solution form, suitable for topical-vaginal administration, for the treatment of vaginal disorders, such as vaginal dryness and vulvovaginal atrophy, wherein the oxygen has a purity ranging between 80% and 97% by volume (v/v).

### Problems of the background art

With respect to the prior art, there is a need to find an association of at least two active substances, which is effective in the treatment of vaginal disorders or diseases or vaginal pathological conditions, such as vulvovaginal atrophy and/or vaginal dryness, and which is able to obtain a more than additive and/or synergistic effect with respect to the action of the single active ingredient. Ozone and hyaluronic acid are both active vaginally; ozone is able to assist and/or amplify the action of hyaluronic acid and vice versa.

In addition to this, it is necessary to find an association of two active substances that is: a simpler solution than the technologies known to the state of the art; cheaper; easily administered, even by a non-medical operator, for example in a domestic context.

### SUMMARY OF THE INVENTION

A first object of the invention is an association comprising
an oxidising and conditioning active ingredient consisting of ozone in gaseous form and
hyaluronic acid,
for use in the topical treatment of vaginal disorders or pathological conditions.

More preferably, the association of the invention is employed in the treatment of vaginal dryness and vulvovaginal atrophy.

A further object of the invention is a kit comprising
- the association for use according to the invention,
- at least one generator of ozone in gaseous form,
- the formulation comprising hyaluronic acid,
- means for applying and/or delivering ozone in gaseous form topically vaginally, preferably at least one cannula.

### Advantages of the invention

The association of the invention is advantageous for the following reasons:
- it is effective in the treatment of vaginal disorders/diseases or pathological conditions, such as, for example, vulvovaginal atrophy or vaginal dryness;
- while being aware that ozone is easily decomposable in a short period of time (about 20 minutes), the Applicant considers that the use of ozone *in gaseous form* directly topically vaginally exerts a more direct and rapid action, preferably in a time span < 20 minutes, during which its decomposition does not occur, or occurs to a minimum extent;
- ozone has a relatively high oxidising power; by virtue of this, ozone in gaseous form, applied topically and directly, has a rapid or faster action compared to other known technologies, for example employing oxygen in gaseous form. Due to the high oxidising power of ozone, the latter could induce the degradation of other active substances or agents, a phenomenon that has not been recorded in the context of the association of the invention;
- ozone is an active conditioning agent, as it is used to carry out an initial conditioning of the vaginal environment, preferably *not simultaneously* with the use of hyaluronic acid, preferably after the use of hyaluronic acid;
- ozone can be obtained by means of an ozone generator, powered at least 100V in alternating current or 12V in direct current, which converts the oxygen present in the *air* (approximately equal to 21% of the total) into ozone by means of at least one electric discharge. The Applicant considers that this ozone generator is less expensive than those generally known on the market, i.e. those based on cylinders *of pure* oxygen converted into ozone. In addition to this, the ozone generator used for the present purposes is more compact and characterised by a reduced weight, so it is also easier to transport.

### DETAILED DESCRIPTION OF THE INVENTION

### Association of the invention

The association (or combination) of active ingredients of the invention comprises or consists of
a first oxidising and/or conditioning active ingredient consisting of ozone in gaseous form and
a second active ingredient consisting of hyaluronic acid,
and is for use in the topical treatment of vaginal disorders or diseases or pathological conditions.

Oxidising active agent is meant as an active ingredient with oxidising action, preferably capable of oxidising the vaginal environment.

Antioxidant active ingredient, which is for example hyaluronic acid, is meant as an active ingredient with antioxidant action, preferably for the vaginal environment.

Conditioning agent is meant as an agent capable of conditioning the vaginal environment, it preferably prepares the vaginal environment for subsequent treatment, i.e. to the action of hyaluronic acid, promoting an oxidising action associated with that of disinfectant and/or microbicide. Ozone, despite not acting simultaneously, is able to enhance the action of hyaluronic acid.

Preferably, the association comprises or consists of
a first oxidising and conditioning active ingredient consisting of ozone in gaseous form and
a formulation comprising, in turn:
   - an antioxidant active ingredient consisting of hyaluronic acid, and
   - suitable excipients and/or diluents.
and is for use in the topical treatment of vaginal disorders or diseases or pathological conditions.

### Ozone in gaseous form

The association comprises a first oxidising and conditioning active ingredient consisting of ozone in gaseous form.

Ozone in gaseous form is preferably generated by an ozone generator operating at a power ranging between 100 and 240 V, preferably between 110 and 240 V, preferably between 200 and 240 V.

It should be noted that the ozone is preferably not in liposomal form or in the form of micro- or nano-liposomes; the ozone comprised in the association of the invention is not in aqueous solution.

Preferably, the ozone generator, in use, converts the oxygen present in the air (about equal to 21%) into ozone. The Applicant assumes that some of the oxygen present in the air may not be converted into ozone. This amount of oxygen not converted into ozone is, however, much less than 21% of starting oxygen. In addition to this, the Applicant is aware that ozone, being unstable, could decompose and turn, in small part, into oxygen. Having said this, the Applicant considers that oxygen in gaseous form, possibly present in combination with ozone in gaseous form, cannot impart a medical effect topically vaginally, since it would be at a percentage too low to obtain a therapeutic effect. Therefore, the Applicant considers that the only real active ingredient in gaseous form included in the association of the invention is ozone in gaseous form. The remaining gases in the air, for example nitrogen, are considered inert.

The flow of ozone in gaseous form is preferably ranging between 10 and 1200 mg/h, preferably between 10 and 1100 mg/h, preferably between 20 and 1000 mg/h, preferably between 50 and 1000 mg/h, preferably between 80 and 1000 mg/h, preferably between 100 and 1000 mg/h, preferably between 200 and 1000 mg/h, preferably between 400 and 1000 mg/h, preferably between 500 and 1000 mg/h, preferably between 800 and 1000 mg/h, preferably between 500 and 1000 mg/h, preferably between 500 and 800 mg/h, preferably between 500 and 600 mg/h, preferably between 400 and 500 mg/h, preferably between 300 and 500 mg/h, preferably between 250 and 350 mg/h, preferably between 200 and 300 mg/h, preferably between 100 and 250 mg/h, preferably between 100 and 200 mg/h, preferably between 100 and 180 mg/h, preferably between 150 and 180 mg/h.

According to a preferred embodiment, the flow of ozone in gaseous form ranges between 150 and 400 mg/h, preferably it is equal to 150 or 400 mg/h.

The concentration of ozone in gaseous form preferably ranges between 10 and 700 ppm (parts per million), preferably between 20 and 700 ppm, preferably between 50 and 700 ppm, preferably between 80 and 700 ppm, preferably between 100 and 700 ppm, preferably between 200 and 700 ppm, preferably between 250 and 700 ppm, preferably between 250 and 600 ppm, preferably between 250 and 500 ppm, preferably between 250 and 400 ppm, preferably between 250 and 350 ppm, preferably between 250 and 300 ppm, preferably between 200 and 250 ppm, preferably between 100 and 200 ppm, preferably between 100 and 150 ppm, preferably between 90 and 150 ppm. According to a preferred embodiment, the concentration of ozone in gaseous form ranges between 90 and 350 ppm, preferably between 90 and 250 ppm or alternatively ranges between 100 and 250 ppm.

The outflow of ozone ranges between 3 and 8 L/min, preferably between 4 and 7 L/min, preferably it is 5 L/min.

The Applicant considers that, ozone being in gaseous form and being an oxidising and/or conditioning active ingredient, ozone oxidises and/or conditions the vaginal environment, therefore *not simultaneously* or *after* the action of the antioxidant active ingredient hyaluronic acid comprised in the association of the invention.

Preferably, the ozone in gaseous form is administered prior to administration of the active ingredient hyaluronic acid.

Preferably, the delivery of ozone in gaseous form takes place in a period of time ranging between 3 and 10 minutes, preferably between 4 and 8 minutes, preferably equal to 6 minutes.

### Hyaluronic acid and formulation comprising hyaluronic acid

The association also comprises a second active ingredient consisting of hyaluronic acid.

Preferably, the hyaluronic acid is comprised in a formulation, the latter not comprising ozone in gaseous form; in other words, the formulation as comprising hyaluronic acid *does not* comprise ozone in gaseous form.

The Applicant has observed that hyaluronic acid is inert to ozone in gaseous form; it does not undergo degradation if it were to be used simultaneously with ozone, preferably in a time span according to the protocol, nor when administered following a first treatment with only ozone.

Preferably, hyaluronic acid is the only ingredient comprised in the formulation, the latter comprising suitable excipients and/or diluents.

Preferably, hyaluronic acid is comprised in a formulation in solid, liquid or semi-solid form.

According to a particularly preferably embodiment, hyaluronic acid is comprised:
- in a solid formulation, preferably a vaginal ovule, or
- in a liquid formulation, preferably a solution.

### Ist embodiment: hyaluronic acid comprised in the solid formulation

Preferably, the hyaluronic acid is comprised in a solid formulation.

The solid formulation, preferably in the form of a vaginal ovule, comprises or consists of:
- the active ingredient consisting of hyaluronic acid, and
- suitable excipients and/or diluents.

The solid formulation is preferably for topical vaginal use. The solid formulation is preferably (in the form of) a vaginal ovule.

Preferably, suitable excipients and/or diluents for the preparation of vaginal ovules are known to the person skilled in the art. For example, water-soluble excipients are often used, which melt at body temperature, but dissolve slowly in vaginal secretions; therefore, they are able to gradually release, in a continuous and controlled manner, the contained active ingredients. Examples of hydrophilic excipients are: glycerinated gelatin; diols (e.g., methylpropanediol, 1,2-Hexanediol and 1,2-Octanediol); polyethylene glycols (PEG) or mixtures of the foregoing.

For the preparation of vaginal ovules, lipophilic excipients, for example of the type used for the preparation of suppositories, may also be preferably employed. These lipophilic excipients also melt at body temperature, but they release the active ingredient quickly and completely, without remaining in contact with the vaginal mucosa for a time from which systemic effects can be potentially derived. Examples of lipophilic excipients are: synthetic and/or semi-synthetic glycerides and/or of vegetable origin, for example mono-, di- and triglycerides esters of fatty acids, preferably with a number of carbon atoms ranging between 8 and 10, preferably saturated fatty acids, with a predominance of the triester fraction; cocoa butter and/or its derivatives.

For the preparation of vaginal ovules, excipients selected from the group consisting of: gelatin, glycerinated gelatin, diols, PEGs and/or their derivatives, synthetic and/or semi-synthetic glycerides and/or of vegetable origin, cocoa butter and/or its derivatives, moisturisers, humectants, dyes, preservatives, pH modifiers, diluents, flavourings, water, polymers that increase mucoadhesion (acrylic acid derivatives, or cellulose derivatives), rheological modifiers, thickeners, stabilizers, gelling agents, and mixtures of the foregoing may preferably be included.

Preferably, the hyaluronic acid comprised in the formulation is in an amount ranging between 0.05% and 1% by weight, preferably between 0.07% and 1% by weight, preferably between 0.1% and 0.5% by weight, preferably between 0.2% and 0.3% by weight, preferably equal to 0.2% by weight, on the total weight of the formulation.

In the solid formulation, the hyaluronic acid preferably has a molecular weight ranging between 100 kDa and 250 kDa, preferably between 180 kDa and 230 kDa.

Preferably, the hyaluronic acid comprised in the formulation is a derivative of hyaluronic acid, preferably an ester derivative of hyaluronic acid, preferably it is an ester of hyaluronic acid with benzyl alcohol; preferably, 50% of the carboxylic groups of hyaluronic acid are esterified with the corresponding hydroxyl groups of benzyl alcohol.

### 2nd embodiment: hyaluronic acid comprised in the liquid formulation

Preferably, the hyaluronic acid is comprised in a liquid formulation.

The liquid formulation, preferably in the form of a solution, comprises or consists of:
- hyaluronic acid active ingredient, and
- suitable excipients and/or diluents.

The liquid formulation is preferably for topical use, preferably it is applied to the outer area of the female genitalia.

Preferably, suitable excipients and/or diluents for the preparation of vaginal solutions are known to the person skilled in the art. For example, excipients/diluents selected from the group consisting of: solvents (water), humectants, conditioners, chelants, antistatic agents, emulsifiers, surfactants, preservatives, buffer systems, pH modifiers, flavourings, deodorants, rheological agents, antioxidants may preferably be included.

Preferably, the excipients and/or diluents are selected from the group consisting of (*INCI names*): aqua, disodium EDTA, PVP (or Polyvinylpyrrolidone), phenoxyethanol, benzoic acid, dehydroacetic acid, ethylhexylglycerin, sodium hydroxide, lactic acid, perfume, benzyl alcohol, methyl benzoate, Butylhydroxytoluene (BHT), PEG-40 hydrogenated castor oil, and mixtures of the foregoing.

Preferably, the hyaluronic acid comprised in the formulation is in an amount ranging between 0.05% and 1% by weight, preferably between 0.07% and 1% by weight, preferably between 0.1% and 0.5% by weight, preferably between 0.2% and 0.3% by weight, preferably equal to 0.2% by weight, on the total weight of the formulation.

In the liquid formulation, the hyaluronic acid preferably has a molecular weight ranging between 40 kDa and 1400 kDa.

According to a preferred embodiment, the liquid formulation comprises: hyaluronic acid at a molecular weight ranging between 40 kDa and 60 kDa, and hyaluronic acid at a molecular weight ranging between 1000 kDa and 1400 kDa.

The amount of water comprised in the liquid formulation ranges between 95% and 98% by weight, preferably between 96% and 98% by weight, on the total weight of the liquid formulation.

The liquid formulation is preferably in the form of a bottle, preferably disposable; preferably, each bottle has a volume ranging between 10 and 20 ml, preferably 15 ml.

### 3rd embodiment: hyaluronic acid comprised in the semi-solid formulation

A semi-solid formulation is preferably in the form of a gel, emulgel, foam, mousse.

Preferably, the hyaluronic acid is comprised in a semi-solid formulation.

The semi-solid formulation, preferably in the form of a gel or mousse, comprises or consists of:
- hyaluronic acid active ingredient, and
- suitable excipients and/or diluents.

The semi-solid formulation is preferably for topical use, preferably it is applied to the outer area of the female genitalia.

Such semi-solid formulation may contain excipients and/or diluents, selected from the group consisting of: chelators, surfactants, emulsifiers, humectants, solubilisers, rheological agents, perfumes, preservatives, buffer systems, pH modifiers, and mixtures of the foregoing.

Preferably, the hyaluronic acid comprised in the formulation is in an amount ranging between 0.05% and 1% by weight, preferably between 0.07% and 1% by weight, preferably between 0.1% and 0.5% by weight, preferably between 0.2% and 0.3% by weight, preferably equal to 0.2% by weight, on the total weight of the formulation.

The Applicant considers that the hyaluronic acid comprised in the formulation exerts its combined effect with that of ozone in gaseous form vaginally *regardless* of the embodiment of the formulation comprising hyaluronic acid.

It should be noted that preferably the association of the invention and the formulation comprising hyaluronic acid described above are not in liposomal form and do not contain liposomes or micro- or nano-liposomes.

Preferably, the association of the invention or the formulation, comprising hyaluronic acid, does not contain: emulsifiers and/or surfactants that are ozonated or in ozonated form; emulsifiers linked (covalently) to hyaluronic acid; vegetable oils or essential vegetable oils in ozonated form.

Ozonated emulsifiers or oils (or in ozonated form) are intended as emulsifiers or oils that have undergone an ozonation process; the latter preferably provides for the passage of the ozone gas through the emulsifier or oil.

### Use of the association of the invention

The association of the invention is for use in the topical treatment of vaginal disorders or diseases or pathological conditions.

Vaginal disorders or diseases are intended to mean disorders or diseases that affect the vaginal cavity, such as, for example, vaginal dryness. Vaginal pathological conditions, on the other hand, are intended to mean real pathologies that affect the vaginal cavity, such as, for example, vulvo-vaginal atrophy.

Preferably, said vaginal disorders or diseases or pathological conditions are selected from the group consisting of: vaginal dryness, vulvovaginal atrophy, and combinations of the foregoing.

Preferably, the association is for use in the treatment of women of childbearing potential, or of menopausal or post-menopausal women. Preferably, the association is for use in the treatment of women aged between 20 and 75 years, preferably between 22 and 70 years.

Preferably, the association is suitable to be used also for women with the condition of vaginal prolapse.

Vaginal prolapse (or Pelvic Organ Prolapse or "POP") is a condition that occurs when the muscles supporting the organs in the pelvis weaken and one or more organs begin to protrude into the vagina and sometimes even outside it. Organs that can prolapse in this way include: the bladder; the uterus; the urethra; the intestinal rectum. The prolapse may involve the anterior and/or posterior vaginal wall. Anterior vaginal wall prolapse is commonly referred to as cystocele or urethrocele (when the bladder or urethra is involved). Posterior vaginal wall prolapse is commonly referred to as enterocele (when the small bowel and peritoneum are involved) and rectocele (when the rectum is involved). Symptoms include a sense of fullness or pelvic or vaginal weight.

Preferably, the symptomatology associated with vaginal dryness is selected in the group consisting of: irritation; itching; burning; pain; pain or slight bleeding during intercourse; reduction in sexual desire; difficulty in reaching orgasm; frequent stimulation or urgency to urinate; frequent appearance of urinary infections or cystitis; mixtures of the foregoing.

Preferably, the symptomatology associated with vulvovaginal atrophy is selected from the group consisting of: vaginal dryness; vaginal burning; vaginal discharge; itching in the intimate parts; burning when urinating; urgency to urinate; presence of other urinary tract infections or cystitis; urinary incontinence; bleeding, preferably light bleeding, during sexual intercourse; uncomfortable sensation during sexual intercourse; reduced lubrication of the vagina; reduction and narrowing of the vaginal canal; thinning of the vaginal mucosa; mixtures of the foregoing.

The female genital apparatus is the complex of organs and anatomical structures, which aims at controlling the mechanism of reproduction, from the production of egg cells to that of sex hormones.

The organs and the anatomical structures located within the apparatus are: the vagina, the cervix, the uterus, the Fallopian tubes and the ovaries. Specifically, the vagina is the fibro-muscular channel that puts the uterus in communication with the outside; it is connected to the cervix, which represents the lowest part of the uterus.

The organs and the anatomical structures located outside, on the other hand, are: the mons veneris, the labia majora, the labia minora, the Bartholin's glands and the clitoris. The latter, as a whole, take the anatomical name of vulva.

### Kit comprising the association of the invention

Kit, preferably kit for use in the topical treatment of vaginal disorders or diseases or pathological conditions, comprising:
- the association of the invention comprising ozone in gaseous form and hyaluronic acid, or the association comprising ozone in gaseous form and the formulation comprising the active hyaluronic acid and suitable excipients and/or diluents,
- at least one generator of ozone in gaseous form,
- the formulation comprising hyaluronic acid, preferably in solid or liquid form, preferably in the form of vaginal ovules or in the form of a solution,
- means for applying and/or delivering ozone in gaseous form topically vaginally, preferably at least one cannula, preferably at least one disposable cannula, preferably at least one disposable cannula with at least one gas outlet hole,
- optionally, connection means, preferably connection means between the at least one generator of ozone in gaseous form and the means for delivering the ozone in gaseous form topically vaginally, for example a cannula,
- optionally, means for regulating the flow of ozone in gaseous form, preferably a flow meter.

It should be noted that the connection means may preferably be pipes in a material suitable for containing a flow of ozone in gaseous form, preferably they are plastic pipes.

### Therapeutic protocol

Preferably, the method according to the invention comprises the following steps:
- delivering the active ingredient ozone in gaseous form topically vaginally or step (a);
- topically applying the active ingredient hyaluronic acid or step (b), preferably topically applying a formulation comprising hyaluronic acid, the formulation preferably being in solid (vaginal ovule) or liquid (solution) form.

Preferably, the method according to the invention comprises the following stages:
- applying at least one lubricating substance, such as for example the same hyaluronic acid, preferably the at least one lubricating substance being comprised in a formulation for topical use, preferably the formulation being in the form of a solution or solid (e.g. cream, ointment, gel, ovule);
- delivering the active ingredient ozone in gaseous form topically vaginally or step (a);
- topically applying the active ingredient hyaluronic acid or step (b), preferably topically applying a formulation comprising hyaluronic acid, the formulation preferably being in solid (vaginal ovule) or liquid (solution) form.

The protocol preferably consists of 5 sessions in total; preferably, *at least* one session per week, preferably one session per week, is made.

### Step (a)

Preferably, step (a) has a duration preferably ranging between 3 and 10 minutes, preferably ranging between 4 and 8 minutes, preferably equal to 6 minutes.

Preferably, step (a) above provides for the delivery of the active ingredient ozone in gaseous form topically vaginally through the use of a suitable delivery means, such as for example a cannula, preferably a cannula comprising at least one gas outlet hole, preferably a disposable cannula.

Preferably, the means suitable for delivering/applying ozone in gaseous form is inserted into the vaginal cavity, preferably starting from the external access of the vaginal opening, gradually entering the interior, until contact with the external os of the uterine cervix. This gradual insertion procedure allows to avoid trauma in conditions of any vaginal prolapse that may be present or occur in some subjects.

In anatomy, external os refers to the intravaginal segment of the cervix. Cone-shaped, with the trunk apex facing downwards, the external ostypically measures 8-12 mm in length. The apex has an orifice (lower orifice of the cervix or external orifice of the uterus) that enters the uterine cavity.

Preferably, the medium suitable for delivering/applying ozone in gaseous form is introduced, rotating the position of the medium so as to deliver ozone in gaseous form over the entire vaginal surface, preferably the rotation is 360 degrees.

### Step (b)

Preferably, step (b) provides for topically applying the active ingredient hyaluronic acid, comprised in the formulation, once the medium suitable for delivering/applying ozone in gaseous form has been removed, preferably once the cannula has been removed.

### EXAMPLES

Below, the Applicant sets out examples for illustrative but not limiting purposes.

### Example 1 - Intravaginal ozone therapy and hyaluronic acid protocol

Below, a therapeutic protocol is described that provides for the application of the association according to the invention (intravaginal ozone therapy associated with hyaluronic acid ovule or, alternatively, with a hyaluronic acid solution). The protocol can be easily followed even in case of self-application or home application.

The protocol provides for 5 sessions in total. One session is performed every week, for a total of 5 weeks. The duration of the treatment is 6 minutes.

For the generation of the flow of ozone in gaseous form, use is made of the device type: TCB-66300A2CLL, which works at a power of AC200-240V. The flow of ozone is variable between 150 and 400 mg/h.

### Example of solid formulation (ovule) based on hyaluronic acid:

Composition of the solid formulation (ovule): slow-release derivative of hyaluronic acid Hydeal-D 0.2%; other excipients and/or diluents: glycerides of vegetable origin, methylpropanediol, 1,2-hexanediol, caprylyl glycol, purified water, lactic acid.

### Example of liquid formulation (solution) based on hyaluronic acid:

| ***Component*** | ***Percentage quantity (%)*** |
|---|---|
| Millipore purified water | 97.53454 |
| Hyaluronic acid sodium salt m.w. 1000 - 1400 kDa | 0.10000 |
| EDTA disodium salt | 0.15000 |
| Hyaluronic acid sodium salt (low) m.w. 40-60 kDa | 0.10000 |
| PVP | 1.00000 |
| Phenoxyethanol | 0.82000 |
| Benzoic acid | 0.13000 |
| Dehydroacetic acid | 0.07500 |
| Ethylhexylglycerin | 0.03000 |
| Sodium hydroxide | 0.02000 |
| Lactic acid | 0.01600 |
| Water | 0.00400 |
| Scent | 0.01000 |
| Phenoxyethanol | 0.00039 |
| Benzyl alcohol | 0.00005 |
| Methyl benzoate | 0.00003 |
| BHT | 0.00010 |
| PEG-40 hydrogenated castor oil | 0.01000 |

### Inclusion criteria

The subjects included in the study suffer from vaginal atrophy, of the menopausal type or not. Some patients take tamoxifen; other patients undergo radiation oncology therapy.

Symptoms reported by subjects under pre-treatment are: pain, burning, dyspareunia, dryness, itching, cystitis.

Initially, a gentle passage with a wadding pad soaked in water is carried out to clean the wall from mucous secretions and any desquamating cells.

Subsequently, a preliminary lubrication can possibly be carried out at the time of application of the cannula delivering ozone in gaseous form.

The therapeutic protocol developed by the Applicant comprises the following stages:
- delivery of the active ingredient ozone in gaseous form topically vaginally or first step (a);
- topical application of the active ingredient hyaluronic acid or second step (b), preferably topical application of a formulation comprising hyaluronic acid, the formulation preferably being in the form of a vaginal ovule or a solution.

### Step (a)

Ozone in gaseous form is delivered by means of a cannula that is connected to the ozone generator. The cannula delivering ozone in gaseous form is inserted into the vaginal cavity, specifically starting from the external access of the vaginal opening, gradually entering the interior, until contact with the external os of the cervix. This gradual insertion procedure allows to avoid trauma in conditions of any vaginal prolapse that may be present or occur in some subjects.

The cannula, delivering ozone in gaseous form, is applied for a time equal to 6 minutes, by rotating the position of the cannula in order to radiate and deliver ozone in gaseous form over the entire vaginal surface at 360 degrees.

Then, after a time equal to 6 minutes of application of ozone in gaseous form has elapsed, the cannula is removed.

It should be noted that this first step, since it involves the delivery of ozone in gaseous form, induces a powerful oxidation of the intravaginal context, and conditions or prepares to the second step (b) below.

### Step (b)

Once the cannula has been removed, a vaginal ovule based on hyaluronic acid is inserted, or the solution is applied to the external genitalia.

### Result evaluation criteria

*Subjective* evaluation criteria: improvement of pain and physical discomfort during sexual intercourse; reduction or disappearance of burning and/or heat.

*Objective* evaluation criteria: cleansing with the wadding pad (pre-treatment with ozone) shows greater hydration of the pad; the latter appears wetter; increase in the overall weight of the pad; lower placards of flaking cells (greyish, furfuraceous, opaque material).

**Table 1 - Clinical cases treated**

| **Subject** | **Age** | **Pathological condition or vaginal disorder or disorder pre-treatment** | **Post-treatment condition** |
|---|---|---|---|
| Subject 1 | 70 | Vulvovaginal atrophy and uterine prolapse | Greater lightness and relief from the burden of the bladder uterine prolapse |
| Subject 2 | 55 | Menopausal atrophy and cystitis | Improved trophism; no more cystitis |
| Subject 3 | 62 | Subject being treated with tamoxifen (breast cancer); severe atrophy because of chemotherapy | Great relief; termination of itching; resumption of sexual intercourse |
| Subject 4 | 58 | High-dose chemotherapy; inflammation with bleeding fissures | Rapid healing |
| Subject 5 | 64 | Menopausal atrophy | Improved atrophy |
| Subject 6 | 68 | Uterine carcinoma with radiotherapy | Greater lightness and less continence |
| Subject 7 | 70 | Uterine carcinoma with radiotherapy; cystitis; sepsis | Signs of chronic inflammation disappeared; |
| | | | Bad odours gone |
| Subject 8 | 52 | Vulvar atrophy; dyspareunia | Freshness and improved sexual desire |
| Subject 9 | 36 | Amenorrhoea; contraceptive abuse | Improved spasms |
| Subject 10 | 22 | Vaginal sub-atrophy; polycystic ovary | Improved sexual activity |
| Subject 11 | 44 | Intolerance due to multiple chemical sensitivities | Fewer allergies |

### Conclusions

The sessions were well tolerated by the subjects who also reported a pleasant sensation. Home self-management was also easy, with simple explanations. At the end of the treatment, there was no unpleasant feeling. The effects of improving the situation vaginally were gradual during the five sessions, regardless of the type of liquid or solid formulation based on hyaluronic acid used.

## Claims

1. Association comprising
an oxidising and conditioning active ingredient consisting of ozone in gaseous form, and
hyaluronic acid,
for use in the topical treatment of vaginal disorders or pathological conditions.

2. Association for use according to claim 1, wherein said vaginal disorders or pathological conditions are selected from the group consisting of: vaginal dryness, vulvovaginal atrophy, and combinations of the foregoing.

3. Association for use according to claim 1 or 2, wherein the ozone flux in gaseous form is ranging between 10 and 1200 mg/h.

4. Association for use according to any one of claims from 1 to 3, wherein hyaluronic acid is comprised in a formulation, the latter not comprising ozone in gaseous form.

5. Association for use according to claim 4, wherein hyaluronic acid is comprised in the formulation in an amount ranging between 0.05% and 1% by weight of the total weight of the formulation.

6. Association for use according to claim 4 or 5, wherein hyaluronic acid is the only active ingredient comprised in the formulation.

7. Association for use according to any one of claims from 1 to 6, wherein hyaluronic acid is comprised in a formulation in solid, liquid or semi-solid form, preferably the formulation is in solid or liquid form.

8. Kit comprising
- the association for use according to any one of claims from 1 to 7,
- at least one generator of ozone in gaseous form,
- the formulation comprising hyaluronic acid according to any one of claims from 4 to 7,
- means for delivering the ozone in gaseous form topically vaginally, preferably at least one cannula.
